(19) Europäisches Patentamt
European Patent Office
Office européen des brevets

(11) EP 1 909 918 B1

(12) EUROPEAN PATENT SPECIFICATION

(45) Date of publication and mention of the grant of the patent:
**04.09.2013 Bulletin 2013/36**

(21) Application number: **06743508.1**

(22) Date of filing: **28.04.2006**

(51) Int Cl.:
*A61Q 17/04* (2006.01) *A61Q 19/00* (2006.01)
*A61Q 19/02* (2006.01) *A61K 8/92* (2006.01)
*A61K 8/37* (2006.01)

(86) International application number:
**PCT/FI2006/000139**

(87) International publication number:
**WO 2006/117430 (09.11.2006 Gazette 2006/45)**

# (54) SKIN LIGHTENING COMPOSITIONS

HAUTAUFHELLUNGSZUSAMMENSETZUNGEN

PRÉPARATIONS POUR L'ÉCLAIRCISSEMENT DE LA PEAU

(84) Designated Contracting States:
**AT BE BG CH CY CZ DE DK EE ES FI FR GB GR HU IE IS IT LI LT LU LV MC NL PL PT RO SE SI SK TR**

(30) Priority: **29.04.2005 FI 20050453**

(43) Date of publication of application:
**16.04.2008 Bulletin 2008/16**

(73) Proprietor: **Aromtech Ltd**
**95410 Tornio (FI)**

(72) Inventors:
- **YANG, Barou**
**FI-20660 Turku (FI)**
- **JUDIN, Vesa-Pekka**
**FI-03100 Nummela (FI)**
- **MÄÄTTÄ; Petri**
**FI-95440 Kyläjoki (FI)**

(74) Representative: **Suominen, Kaisa Liisa**
**Turun Patenttitoimisto Oy**
**P.O.Box 99**
**20521 Turku (FI)**

(56) References cited:

| | |
|---|---|
| **EP-A- 1 454 622** | **WO-A-97/46219** |
| **WO-A-99/55352** | **WO-A-2004/060364** |
| **WO-A-2004/082642** | **DE-A1- 10 163 786** |
| **FR-A- 2 720 901** | **US-A1- 2002 022 006** |
| **US-A1- 2002 168 430** | **US-A1- 2005 048 020** |

- **PATENT ABSTRACTS OF JAPAN vol. 2003, no. 12, 5 December 2003 (2003-12-05) -& JP 2004 123563 A (ISKRA IND CO LTD), 22 April 2004 (2004-04-22)**
- **PATENT ABSTRACTS OF JAPAN vol. 014, no. 314 (C-0737), 5 July 1990 (1990-07-05) -& JP 02 108613 A (KUROODA JAPAN KK), 20 April 1990 (1990-04-20)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 909 918 B1

## Description

### FIELD OF THE INVENTION

The present invention relates to a use of a composition comprising a seed oil comprising triglycerides of unsaturated fatty acids and a skin care composition for skin lightening.

### BACKGROUND OF THE INVENTION

Hyperpigmentation, chloasmata and senile spots are signs of ageing of the skin and ultraviolet (UV) exposure often results in hyperpigmentation, dyspigmentation, and uneven skin tones. In Asian, African and Hispanic cultures there is also a strong desire of lighter skin tones. There is therefore a great demand for ingredients and composition for skin lightening formulations and for formulations evening the skin tones. Moreover, moisture loss and increased skin sensitivity are often associated with sun/UV exposure and dryness and wrinkles are typical of aged skin.

Sunscreens such as titanium dioxide, zinc oxide and avobenzone are widely used to prevent hyper- and dyspigmentation caused by UV exposure. These compounds however present a significant risk of skin penetration.

The commonly used skin lightening ingredients are hydroquinone and kojic acid as inhibitors of melanin synthesis. In addition to some safety concerns, such as cancer risk and skin irritation, these ingredients are extremely unstable and lose their activity in the end product soon after opening of the package. To overcome the problem, some derivatives of these compounds such as kojic dipalmitate have been used. However the efficacy of these derivatives has not been fully demonstrated. All trans-retinoic acid, alpha hydroxy acid and hydroxy acids from synthetic and natural sources are not effective in reducing melanin synthesis but are often used in combination with other lightening ingredients to improve the overall appearance of skin.

Document EP 992 236 (published also in Imanaka Hiromichi, Ando Hideya, Makino Taketoshi: Skin-Lightening Cosmetic) discloses the use of polyunsaturated fatty acids with 18 - 22 carbons in the form of free acids and simple derivatives. These compounds are however known to be strong skin irritants. In addition to safety concerns, incorporation of acidic ingredients into a formulation often brings a limitation to the use of some other ingredients due to change in pH. This document also excludes the use of triglycerides of unsaturated fatty acids.

In the present state of the art, treatment of hyper- and dyspigmentation sometimes involves combined use of inhibitors of melanin synthesis and resurfacing measures such as chemical peels and laser treatment. These measures may damage the skin barrier system and result in moisture loss and an increased sensitivity of skin.

Moreover, it is often not enough for sun care products to include only skin lightening ingredients. Other ingredients with moisturising and antioxidative activities are necessary due to the moisture loss and oxidative stress caused by sun exposure.

### OBJECTS AND SUMMARY OF THE INVENTION

The present invention seeks to solve at least part of the problems of the prior art listed above. One object of the present invention is thus to provide a lightening skin care composition that is safe for the user. Another object is to provide an effective and natural skin care composition for skin lightening. The present invention also has an object to provide a skin care composition that is useful as a skin lightening, a skin moisturising and an antioxidant composition.

The invention is characterised in what is defined in the independent claims.

The present invention relates to the use of composition comprising at least one triglyceride of at least one unsaturated fatty acid for manufacture of a product for skin lightening as defined in claim 1.

Some embodiments relates also to a skin care composition comprising a dermatologically acceptable additive and a pure seed oil derived from a plant selected from the group *Vaccinium, Oxycoccus, Fragaria, Hippophae, Echium, Hordeum, Avena,* or a mixture thereof comprising at least one triglyceride of an unsaturated fatty acid.

### BRIEF DESCRIPTION OF THE DRAWING

Fig. 1 shows results of Example 6,

Fig. 2 shows results of Example 6,

Fig. 3 shows results of Example 6,

Fig. 4 shows results of Example 6, and

Fig. 5 shows results of Example 7.

DETAILED DESCRIPTION OF THE INVENTION

The present invention relates to a use of a composition according to claim 1 comprising at least one triglyceride of at least one unsaturated fatty acid for manufacture of a product for skin lightening.

Unsaturated fatty acids in the form of triglycerides have been previously assumed to be inactive in skin care products. The present inventors have however surprisingly discovered that some special vegetable oils extremely rich in triglycerides of unsaturated fatty acids are active ingredients for skin care compositions intended for lightening, smoothing or whitening the skin colour of human objects. It is assumed, without being bound by the theory, that the triglycerides of the unsaturated fatty acids may reduce the biosynthesis of melanin resulting in a lighter skin tone and/or may help to disperse the melanin pigment and thus even the skin tone.

According to an embodiment of the present invention, the said at least one unsaturated fatty acid is selected from the group consisting of linoleic acid and α-linolenic acid. The inventors have found that these two unsaturated fatty acids in the form of triglycerides have a significant skin lightening effect. A mixture of triglycerides of these two acids can also be used. In the present context, linoleic acid does not comprise conjugated linoleic acids, which are structurally different fatty acids and also from different sources, such as from dairy products and synthetic sources.

Oils useful in manufacturing products for lightening and moisturising of the skin would typically contain a total content of linoleic acid [18:2(n - 6)] and α-linolenic acid [18:3(n - 3)] at levels of 20 - 90 %, preferably 60 - 9 0 %, of the total fatty acids. One preferred fatty acid composition of the oils contains linoleic and α-linolenic acids at ratios from 2: 1 to 1:2. According to one embodiment, said ratio is around 1:1. Other ratios may naturally also be used.

General structure of linoleic acid is shown below:

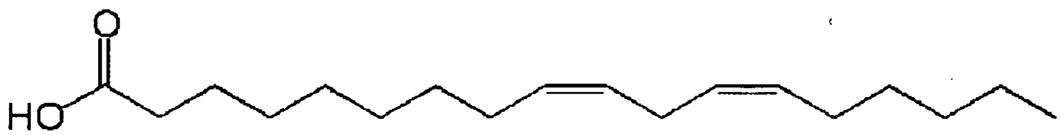

General structure of α-linolenic acid is shown below:

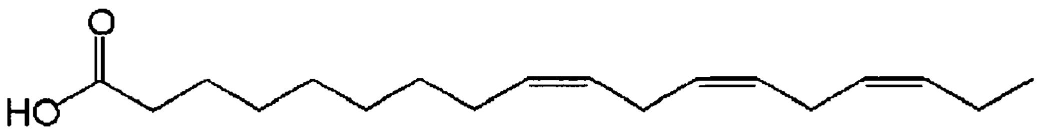

According to the invention triglyceride of unsaturated fatty acid is derived from natural oils of plant origin. The plant can be selected from the group consisting of *Vaccinium, Oxycoccus, Fragaria, Hippophae, Echium, Hordeum* and *Avena.* The natural oil can be a seed oil of *Vaccinium vitis-idaea.* A beneficial composition of triglycerides of these acids can be found, for example, in the seed oils of the above mentioned plants. In some cases, e.g. in relation with genus *Hippophae,* a fruit pulp oil can be used. Also mixtures of these natural oils can be used. Mixture can comprise oils from two or more different plant genera, oils from two or more different plant species of the same genus or oils from seeds and fruit pulp, or any possible combination of these.

According to one embodiment of the invention the seed oil, which is used for skin lightening, is derived from seeds of a Vaccinium species that is selected from the group comprising species of blueberry, e.g. *Vaccinium angustifolium, Vaccinium boreale, Vaccinium caesariense, Vaccinium corymbosum, Vaccinium darrowii, Vaccinium elliottii, Vaccinium formosum, Vaccinium fuscatum, Vaccinium hirsutum, Vaccinium koreanum, Vaccinium myrsinites, Vaccinium myrtilloides, Vaccinium pallidum, Vaccinium simulatum, Vaccinium tenellum, Vaccinium virgatum;* species of bilberry, e.g. *Vaccinium myrtillus, Vaccinium uliginosum L., Vaccinium caespitosum, Vaccinium deliciosum, Vaccinium membranaceum, Vaccinium ovalifolium;* cowberry and lingonberry, i.e species of Vaccinium vitis-idaea; species of cranberry, e.g. *Vaccinium macrocarpon, Vaccinium microcarpum, Vaccinium oxycoccus.* Especially oil from lingonberry can be used.

According to one embodiment oil from species of *Hippophae*, e.g. *Hippophae rhamnoides, Hippophae salicifolia, Hippophae tibetana,* can be used.

In this context by skin care compositions it is meant for example cosmetic and dermatological compositions.

One embodiment of the present invention thus provides a skin care composition that is a natural skin-lightening agent with no skin- and membrane-irritating effects or other health risks. The compositions are useful for example in formulations for sensitive skin type or for prolonged or continuous use. Moreover, skin care compositions according to the present invention can be used for multiple purposes, for example at the same time for skin lightening, moisturising and anti-ageing purposes due to the surprisingly multiple functions of the seed oils used.

The skin care composition according to the present invention comprises natural plant oil as defined by claim 4 comprising at least one triglyceride of at least one unsaturated fatty acid. According to a preferred embodiment, one triglyceride comprises more than one type of unsaturated fatty acid.

According to another embodiment of the present invention, the amount of the natural seed oil, is 0.01 - 99.99, typically 0.05 - 50, more typically 0.1 - 25, most typically 1 - 10 weight-% of the weight of the composition. As the oils of the selected plants are of natural origin, they can be used in relatively high concentrations without any adverse effects. On the other hand the natural plant oils have beneficial triglyceride compositions that are potent in skin lightening, thus making the amount of needed oil relatively small.

Triglycerides of unsaturated fatty acids derived from the seed oils, mentioned above have been found out to be effective for example for lightening for evening skin tones. More specifically, triglycerides of unsaturated fatty acids derived from seed oils of *Vaccinium* (e.g. bilberry, blueberry, lingonberry, mountain bilberry, cranberry and arctic cranberry), *Oxycoccus* (e.g. cranberry), *Fragaria* (e.g. strawberry), *Hippophaë* (e.g. sea buckthorn), *Echium* (e.g. echium), *Hordeum* (e.g. barley) and *Avena* (e.g. oat) have been found to be especially effective for lightening or evening skin tones.

In addition, these oils are effective for moisturising skin and for restoring the skin barrier antioxidation and antiageing. Triglycerides of unsaturated fatty acids derived from seed oils of *Vaccinium* (e.g. bilberry, blueberry, lingonberry, mountain bilberry, arctic cranberry), *Oxycoccus* (e.g. cranberry), *Fragaria* (e.g. strawberry), *Hipphophaë* (e.g. sea buckthorn), *Echium* (e.g. echium), *Hordeum* (e.g. barley) and *Avena* (e.g. oat) have been found to be especially effective for moisturising skin and restoring the skin barrier. Furthermore seed oils rich in triglycerides of unsaturated fatty acids, e.g. seed oils from *Vaccinium* (e.g. bilberry, blueberry, lingonberry, mountain bilberry, arctic cranberry), *Oxycoccus* (e.g. cranberry) and *Echium* (e.g. echium) are effective for antioxidation and anti-ageing of skin. Thus the skin composition may show other functions including moisturising and anti-ageing properties on top the skin lightening properties.

The skin care compositions according to the present invention is, according to one embodiment of the present invention, intended for skin lightening and may also include a use selected from the group of functions consisting of moisturising, restoring skin barrier, antioxidation, antiageing and mixtures thereof.

In the present context pure seed oil denotes oil comprising at least 95 weight-% seed oil, typically at least 99 weight-% seed oil, more typically 99.5 weight-% seeed oil, sometimes even 100 weight-% seed oil. Pure seed oil is totally solvent free and it does not contain any solid content or solid residues.

The skin care compositions of the present invention can be used for example in day creams, night creams, face and body lotions, body butters, skin peels, masks, and shower gels. The targets of these products can be for example lightening, moisturising, anti-ageing and creams and lotions for after sun application.

According to one embodiment of the invention the the dermatologically acceptable additive comprises other lightening ingredients, sunscreen ingredients, preservatives, antioxidants or their combinations. The additive may be also emulsifier, stabilizer, preservative, UV filter, emollient, organic or mineral fillers. Typically the skin care composition is solvent free, thus making it very suitable for cosmetic purposes and minimising or even eliminating the risks of hypersensitivity or toxicity.

As it is well known to a person skilled in the art, any other additives useful in skin care formulations may be used, such as kojic acid, hydroquinone, arbutin, free fatty acids, honey etc at safe levels. These compounds are typical examples of skin lightening ingredients.

According to yet another embodiment, said triglycerides of polyunsaturated fatty acids are used in the form of natural seed oils of plant origin as defined in claim 1 for lightening the skin tone. They may be used in combination with other skin lightening ingredients and sunscreens to produce enhanced lightening and UV protection efficacies on skin or to reduce the amount of other lightening ingredients and sunscreens in skin care formulations. Some examples of sunscreen ingredients are titanium dioxide and zinc oxide.

Among the different oil manufacturing technologies, supercritical $CO_2$ extraction often has good quality due to the high extraction efficiency and the absence of harmful solvent residues in the oils extracted. Thus supercritical $CO_2$-extracted seed oils are used in for the skin care composition according to claim 4.

The details and embodiments described above in connection with the skin care composition also apply to the use according to the present invention, and vice versa. According to some embodiments, said skin care compositions further comprise lightening and/or sun screen ingredients and/or added antioxidants.

One application of the present invention covers the use of natural oils of plant origin rich in triglycerides of polyunsaturated fatty acids and as active ingredients in skin care formulations for skin lightening as defined in claim 4.

The skin composition according to the present invention can be applied regularly, e.g. once or twice a day. It can also be applied as a limited regime, lasting e.g. for a one week, two weeks, three weeks, four weeks, one month or two months etc.

The skin care composition is useful in whitening or lightening the overall skin tone, or whitening or lightening age spots or freckles. As the skin care composition of the present invention is safe to use, it can be used for lightening of the skin of the whole body, not only face and/or hands. Furthermore, it can be used to lighten or whiten the skin of children or adolescents.

EXPERIMENTAL PART

**Example 1**

An emulsion cream composition for facial and throat skin care contains, in weight-%
cyclopentasiloxane 6.0 wt-%
steareth-2 4.0 wt-%
steareth-21 2.0 wt-%
octyldodecanol 2.0 wt-%
stearic acid 2.0 wt-%
cetearyl alcohol 1.5 wt-%
peg-30 dipolyhydroxystearate 1.0 wt-%
tocopheryl acetate 1.0 wt-%
Vaccinium oxycoccos (Oxycoccus palustris), seed oil 1-10%
phenoxyethanol 0.80 wt-%
ppg-15 stearyl ether 0.60 wt-%
panthenol 0.50 wt-%
perfume 0.20 wt-%
dimethicone/ vinyldimethicon crosspolymer 0.30 wt-%
phospholipids 0.2 wt-%
parabens 0.30 wt-%
magnesium ascorbyl phosphate 0.10 wt-%
tocopherol 0.05 wt-%
glycerin 0.025 wt-%
ascorbyl palmitate 0.05 wt-%
sodium chloride 0.003 wt-%
ascorbic acid 0.001 wt-%
water in order to arrive at 100 wt-%

The described emulsion cream composition lightens, moisturizes, brightens and softens the skin. In addition it has antioxidative properties.

**Example 2**

A ready-for-use emulsion cream composition for facial and throat skin care consists of (in weight-%):

sorbitan olivate 14.0 wt-%
squalane 11.0 wt-%
cyclopentasiloxane 8.0 wt-%
glycerin 4.0 wt-%
tocopheryl acetate 2.0 wt-%
sodium chloride 1.5 wt-%
C20-C40 alcohols 1.0 wt-%
Ribes nigrum, seed oil 1-5%
Vaccinium myrtillus, seed oil 0.01-10 wt-%
C20-C40 acid 0.85 wt-%
phenoxyethanol 0.72 wt-%
aluminium/magnesium hydroxide stearate 0.70 wt-%
dimethicone/vinyldimethicone crosspolymer 0.30 wt-%
perfume 0.20 wt-%
parabens 0.30 wt-%

polyethylene 0.15 wt-%
phospholipids 0.15 wt-%
retinol 0.05 wt-%
tocopherol 0.05 wt-%
ascorbyl palmitate 0.005 wt-%
ascorbic acid 0.005 wt-%
citric acid 0.001 wt-%
retinyl palmitate 0.00001 wt-%
water in order to arrive at 100 wt-%

The emulsion cream composition lightens skin tone, regenerates and revitalises skin. In addition, it reduces, softens and smoothes the fine lines in the skin.

**Example 3**

An emulsion cream composition for intensive lightening and moisturising of facial and throat skin contains, in weight-%
cyclopentasiloxane 6.0 wt-%
steareth-2 4.0 wt-%
steareth-21 2.0 wt-%
octyldodecanol 2.0 wt-%
stearic acid 2.0 wt-%
cetearyl alcohol 1.5 wt-%
peg-30 dipolyhydroxystearate 1.0 wt-%
tocopheryl acetate 1.0 wt-%
Vaccinium vitis-ideae, seed oil 1-10 wt-%
Vaccinium oxycoccos (Oxycoccus palustris), seed oil 1-10%
honey 1 %
phenoxyethanol 0.80 wt-%
panthenol 0.50 wt-%
perfume 0.35 wt-%
dimethicone/ vinyldimethicon crosspolymer 0.28 wt-%
phospholipids 1.0 wt-%
parabens 0.30 wt-%
magnesium ascorbyl phosphate 0.50 wt-%
hydroquinone 0.5%
kojic dipalmitate 0.5%
titanium dioxide (ultra fine) 1.0%
tocopherol 0.05 wt-%
glycerin 0.025 wt-%
ascorbyl palmitate 0.01 wt-%
sodium chloride 0.003 wt-%
ascorbic acid 0.001 wt-%
water in order to arrive at 100 wt-%

**Example 4**

A skin lightening, moisturising, radical scavenging and regenerating body butter for anti-ageing and for after sun treatment of the skin consists of (in weight-%)
Hydrogenated vegetable oil 17 wt-%
Sodium stearoyl lactylate 0.3 wt-%
Glyceryl stearate 0.9 wt-%
Cetearyl alcohol 1.5 wt-%
Vacciniun macrocarpon, seed oil 0.001 - 15 wt-%
Rubus idaea, seed oil 0.01 -10 wt-%
Soybean phospholipids 2 wt-%
C20-C40 fatty alcohols 1.0 wt-%
C20-C40 fatty acids 1.0 wt-%

Glycerin 4 wt-%
Sodium hydroxide 0.2 wt-%
Isoparaffin 2 wt-%
Isodecyl neopentanoate 3 wt-%
Ascorbyl palmitate 0.5 wt-%
Ascorbic acid 0.2 wt-%
α-tocopherol 0.5 wt-%
Phenoxyethanol 0.13 wt-%
Methylparaben 0.13 wt-%
Butylparaben 0.13 wt-%
Etheylparaben 0.13 wt-%
Propylparaben 0.13 wt-%
Isobutylparaben 0.13 wt-%
Water in order to arrive at 100 % by weight.

**Example 5**

A lightening body care formulation with soothing and moisturising efficacies containing by weight-%
cyclopentasiloxane 2.0 wt-%
steareth-2 2.0 wt-%
steareth-21 1.0 wt-%
soybean phospholipids 2.0%
octyldodecanol 2.0 wt-%
stearic acid 2.0 wt-%
cetearyl alcohol 1.5 wt-%
peg-30 dipolyhydroxystearate 1.0 wt-%
tocopheryl acetate 1.0 wt-%
Hordeum vulgare, seed oil 1-10 wt-%
Avena sativa, seed oil 1-10%
Echium plantagineum, seed oil, 1-10%
phenoxyethanol 0.80 wt-%
panthenol 0.50 wt-%
perfume 0.35 wt-%
dimethicone/ vinyldimethicon crosspolymer 0.28 wt-%
phospholipids 1.0 wt-%
parabens 0.30 wt-%
magnesium ascorbyl phosphate 0.10 wt-%
tocopherol 0.05 wt-%
glycerin 0.025 wt-%
ascorbyl palmitate 0.01 wt-%
sodium chloride 0.003 wt-%
ascorbic acid 0.001 wt-%
water in order to arrive at 100 wt-%

**Example 6**

Samples of a cream at 5 wt-% (of the total composition) RED ALFA lingonberry (*Vaccinum vitis-idaea*) seed oil having the following composition (the components are given as weight-%) were tested.

| | |
|---|---|
| Water | 69.30 |
| Hydrogenated polydecene | 20.00 |
| Vaccinum vitis-idaea seed oil | 5.00 |
| Steareth-2 | 3.00 |
| Steareth-21 | 1.00 |
| Cetearyl alcohol | 1.50 |
| Propylene glycol, diazolidinyl urea, methylparaben, propylparaben | 0.20 |

The aim of the study was to evaluate the skin lightening efficacy of a cream containing an active ingredient on skin with dark spots (lentigo senile, melasma) in comparison to a placebo cream. The main targets of such alterations are photoexposed body sites such as face, neck and dorsal parts of hands of people over 25 years old.

12 volunteers, all females, (aged from 30 to 65 years old, average age 52 years), with facial and hand ageing spots applied the active cream daily on one hand or on half face for 60 days. The other half face/hand was treated with the placebo cream and served as a control site. Instrumental evaluation of colorimetry was performed on age spots and on nonspotted "healthy skin" at the beginning (To), after 30 days ($T_{30}$) and after 60 days ($T_F$) of treatment.

Evaluation of the results

Chroma-Meter CR 300 was used for colorimetry. The colour rating system used to read the results is L* a* b*, wherein L* indicates the luminosity of the colours and a* and b* indicate the axis of two colours: a* red/green colour and b* yellow/blue colour.

The following parameters are sensible indexes of changes in pigmentation intensity and were considered in this study:

- L*, which expresses the changes in brightness
- b*, which expresses the colour on the yellow-blue axis
- ITA°, which expresses the melanin index, calculated from L* and b*, according to the following mathematical expression:

$$ITA° = \{Arc.tang.\ (L^*-50)/b^*)\}\ x\ 180/3{,}1416$$

ITA° value is inversely correlated to the pigmentation intensity: the higher the value, the lighter the colour. The whitening efficacy is shown by increased ITA° values and L* values.

Method of performance

The study, designed in blind, was carried out on 12 women who had at least two age spots located symmetrically on each hand and on each half face. Volunteers were asked not to apply any product on the tested areas (hands or face) for at least 3 hours before performing the measurements. At the beginning of the study two spots, one on the right hand/half face and one on the left hand/half face, were carefully selected and their location reported.

A control of uninvolved (nonspotted) healthy skin area on each hand/half face was also selected.

Each volunteer was asked to apply the active cream, twice a day, to one hand/half face, randomly right or left, for 60 days. The other half face/hand was treated with the placebo cream and served as a control site. Instrumental evaluation of skin colorimetry was performed on the selected areas, at the beginning (To), after 30 days of treatment ($T_{30}$) and at the end of the study ($T_F$).

A mathematical elaboration was also used. The values related to each parameter (L* and ITA°) at each check time were mathematically analysed in order to calculate the average and the standard deviation. Furthermore, it was calculated:

$$T_x - T_0 = \text{variation of the parameter}$$

wherein:

$T_x$ = value after 30 days or after 60 days of treatment
$T_0$ = basal value at the beginning of the treatment

This difference is also reported as percentage of variation.

The values recorded at each check and the variations between the initial and the values $T_{30}$ - $T_0$ and $T_F$ - $T_0$ obtained in the active and in the placebo area were statistically compared by means of Analysis of Variance and Tukey test. The groups of data were considered significantly different for a probability value $p \leq 0.05$.

Results

*L* parameter*

Spotted skin:

A statistically highly significant increase in the L* values was found in the site treated with active cream after 30 and 60 days. No significant variation was recorded in the site treated with placebo cream. The results are shown in Tables 1 and 2.

Table 1

| Spotted skin | $T_0$ | $T_{30}$ | $T_f$ | $T_{30}$ - $T_0$ | $T_f$-$T_0$ |
|---|---|---|---|---|---|
| Active | 57.70 ±3.34 | 60.90 ±3.45 | 61.04 ± 2.81 | 3.20 (5.5 %) | 3.34 (5.8 %) |
| Placebo | 58.39 ± 3.06 | 59.49 ± 2.57 | 60.01 ± 3.04 | 1.10 (1.9 %) | 1.62 (2.8%) |

The statistical comparison between the two treatments showed a significant difference of L* values at the comparison $T_{30}$ - $T_0$ and $T_{60}$ - To, as shown in Table 2.

Table 2

| Spotted skin: Tukey-Test | |
|---|---|
| Active | $T_0$ vs $T_{30}$ $p < 0.001$<br>$T_0$ vs $T_F$ $p < 0.001$ |
| Placebo | $T_0$ vs $T_{30}$ $p > 0.05$<br>$T_0$ vs $T_F$ $p > 0.05$ |
| Variation | $T_{30}$ : active vs placebo $p < 0.01$<br>$T_F$ : active vs placebo $p < 0.05$ |

Healthy skin (nonspotted area of skin):

A statistically significant increase in the L* values was found in the site treated with active cream after 30 and 60 days. No significant variation was recorded in the site treated with placebo cream. The results are shown in Tables 3 and 4.

Table 3

| Healthy skin | $T_0$ | $T_{30}$ | $T_f$ | $T_{30}$ - $T_0$ | $T_f$ - $T_0$ |
|---|---|---|---|---|---|
| Active | 60.30 ± 3.19 | 62.55 ± 2.68 | 62.97 ± 2.34 | 2.25 (3.7 %) | 2.67 (4.4 %) |
| Placebo | 60.65 ± 3.08 | 61.76 ± 2.82 | 62.18 ± 1.98 | 1.11 (1.8 %) | 1.53 (2.5 %) |

The statistical comparison between the two treatments did not show any significant difference of L* values at the comparison $T_{30}$ - $T_0$ and $T_{60}$ - To, as shown in Table 4.

Table 4

| Healthy skin: Tukey-Test | |
|---|---|
| Active | $T_0$ vs $T_{30}$ $p < 0.01$<br>$T_0$ vs $T_F$ $p < 0.01$ |
| Placebo | $T_0$ vs $T_{30}$ $p > 0.05$<br>$T_0$ vs $T_F$ $p > 0.05$ |
| Variation | $T_{30}$ : active vs placebo $p > 0.05$<br>$T_F$ : active vs placebo $p > 0.05$ |

The results are also illustrated in Figures 1 and 2. Figure 1 shows the variations in L* values recorded on spotted skin, wherein the active ingredient is shown as the left column and the placebo as the right column. Time is shown in abscissa and L* value in ordinate. Figure 2 shows the same results for healthy skin.

*ITA° parameter*

Spotted skin:

A statistically highly significant increase in the ITA° values was found in the site treated with active cream after 30 and 60 days. A significant variation was recorded in the site treated with placebo cream only at the final check after 60 days. The results are shown in Tables 5 and 6.

Table 5

| Spotted skin | $T_0$ | $T_{30}$ | $T_f$ | $T_{30}$-$T_0$ | $T_f$ -$T_0$ |
|---|---|---|---|---|---|
| Active | 21° ± 7.8 | 30° ± 7.5 | 30° ±7.5 | 9° | 9° |
| Placebo | 24° ±8.8 | 27° ± 7.9 | 29° ± 6.6 | 3° | 5° |

The statistical comparison between the two treatments showed a significant difference of ITA° values at the comparison $T_{30}$ - $T_0$ and $T_{60}$ - To, as shown in Table 6.

Table 6

| Spotted skin: Tukey-Test | |
|---|---|
| Active | $T_0$ vs $T_{30}$ $p < 0.001$<br>$T_0$ vs $T_F$ $p < 0.001$ |
| Placebo | $T_0$ vs $T_{30}$ $p > 0.05$<br>$T_0$ vs $T_F$ $p < 0.05$ |
| Variation | $T_{30}$ : active vs placebo $p < 0.01$<br>$T_F$ : active vs placebo $p = 0.01$ |

Healthy skin:

A statistically highly significant increase in the ITA° values was found in the site treated with active cream after 30 and 60 days. A significant variation was recorded in the site treated with placebo cream only at the final check after 60 days. The results are shown in Tables 7 and 8.

Table 7

| Healthy skin | $T_0$ | $T_{30}$ | $T_f$ | $T_{30}$-$T_0$ | $T_f$ -$T_0$ |
|---|---|---|---|---|---|
| Active | 28° ± 7.8 | 32° ± 6.5 | 36° ± 5.4 | 5° | 8° |
| Placebo | 29° ± 7.5 | 31° ± 6.7 | 33° ± 6.1 | 2° | 4° |

The statistical comparison between the two treatments showed a significant difference of ITA° values only at the comparison $T_{60}$ - To, as shown in Table 8.

Table 8

| Healthy skin: Tukey-Test | |
|---|---|
| Active | $T_0$ vs $T_{30}$ $p < 0.01$<br>$T_0$ vs $T_F$ $p < 0.001$ |
| Placebo | $T_0$ vs $T_{30}$ $p > 0.05$<br>$T_0$ vs $T_F$ $p < 0.05$ |

(continued)

| Healthy skin: Tukey-Test | |
|---|---|
| Variation | $T_{30}$ : active vs placebo $p > 0.05$<br>$T_F$ : active vs placebo $p < 0.05$ |

The results are also shown in Figures 3 and 4, wherein Figure 3 shows the changes in ITA° values recorded on spotted skin. The left columns show the results for the active ingredient and the right columns the results for placebo. Time is shown in abscissa and ITA° in ordinate. Figure 4 shows the same results for healthy skin.

Conclusions

The colorimetric measurements performed on age spots and on "healthy skin" without spots at the beginning, after 30 and 60 days of treatment, showed the following results:

*Spotted skin:*

- A statistically highly significant increase in the L* values in the site treated with active cream after 30 and 60 days. No significant variation in the site treated with placebo cream.

- A statistically highly significant increase in the ITA° values in the site treated with active cream after 30 and 60 days. A significant variation in the site treated with placebo cream only after 60 days.

The statistical comparison between the two treatments showed a significant difference of L* and ITA° values at the comparison $T_{30}$ - $T_0$ and $T_{60}$ - $T_0$.

*Healthy skin:*

- A statistically significant increase in the L* values in the site treated with active cream after 30 and 60 days. No significant variation in the site treated with placebo cream.

The statistical comparison between the two treatments did not show any significant difference of L* values at the comparison $T_{30}$ - $T_0$ and $T_{60}$ - $T_0$.

- A statistically highly significant increase in the ITA° values in the site treated with active cream after 30 and 60 days. A significant variation the site treated with placebo cream after 60 days.

The statistical comparison between the two treatments showed a significant difference of ITA° values only at the comparison $T_{60}$ - $T_0$.

**Example 7**

A comparative study of the skin moisturization potential of RED ALFA lingonberry seed oil at 5 wt-% was carried out via measurements of the skin electrical capacitance (corneometry). This was a clinical double-blind randomized comparative placebo-controlled study, wherein a test cream containing 5% red alfa lingonberry seed oil was compared to a base cream (placebo).

The study was carried out with 20 female volunteers in the 19-60 age range (mean age: 35) of different races and skin types. The exclusion criteria for their selection were: dermatological diseases, pregnancy and breastfeeding.

Corneometry measurements were made with the Corneometer CM 820, The measurement area was 49 $mm^2$. Twenty measurements were made on each area.

The readings indicated the degree of moisture on the skin surface based on variations in electric capacitance. The apparatus scale is arbitrary, i.e., greater reading values indicate greater moisture.

*First stage*

The volunteers stayed in rest for 30 minutes, in an acclimatized room with temperature 20 ± 2 °C and relative humidity 50 ± 5 %.

Symmetrical areas of 25 $cm^2$ of the skin on the anterior area of the legs or arms were chosen at random and demarcated: an area for the application of the test product (base cream + 5 wt-% red alfa lingonberry seed oil), an area for the application of the placebo (base cream, i.e. the same formula without the active ingredient RED ALFA lingonberry seed oil) and an area to be kept as a negative control (i.e. an area without product).

Before the application of the product or the placebo the electric capacitance of each area was obtained from the arithmetic mean of twenty measures:

*Second stage*

0,05 g of the test product (base cream + 5 wt-% RED ALFA lingonberry seed oil) and 0,05 g of the placebo (base cream only) were applied on their corresponding previously demarcated areas on each volunteer. No product was applied on the negative control area. The electric capacitance values of the areas were measured after 60, 120, 180 and 360 minutes, while the volunteers stayed in rest in an acclimatized room. The final value for each area was obtained from the arithmetic mean of twenty measures.

Twenty volunteers completed the study. This corresponds to 100 % of the initial group. No volunteer had any kind of adverse reactions in the areas where the products were applied.

*Results*

Exploratory data analysis was performed. The softwares SAS System for Windows version 8.0 and Minitab for Windows 13.32 were used to perform the statistical analysis. The comparative analysis was accomplished via Student t Test.

Figure 5 shows the increase in the capacitance of skin, indicating increases in skin moisture after the treatment. Time after application is shown in abscissa and the increase in capacitance compared to baseline, after treatment in ordinate. The leftmost column represents the results for the areas treated with base cream containing 5 % of RED ALFA lingonberry seed oil, the middle column represents the results for the areas treated with base cream only, and the rightmost column the results for the untreated areas.

Both the test cream containing 5 wt-% RED ALFA lingonberry seed oil and the placebo (the base cream) caused the moisture content of the skin to increase. Statistically significant difference was found between the test cream containing lingonberry seed oil and the placebo (base) cream. Treatment with the test cream led to a superior increase in the mean capacitance value of the skin (skin moisture) compared to the base cream.

## Claims

1. Use of composition comprising a seed oil derived from a plant selected from the group *Vaccinium, Oxycoccus, Fragaria, Hipphophae, Echium, Hordeum, Avena,* or a mixture thereof, comprising at least one triglyceride of at least one unsaturated fatty acid for skin lightening.

2. Use according to claim 1, **characterised in that** the natural seed oil is seed oil of *Vaccinium vitis-idaea.*

3. Use according to any of the previous claims, **characterised in that** the composition is a day cream, a night cream, a face lotion, a body lotion, a body butter, a skin peel, a mask, a shower gel, a sun cream, a sun lotion, an after sun cream or an after sun lotion.

4. A skin care composition for skin lightening comprising

   - a dermatologically acceptable additive, and
   - a pure seed oil derived from a plant selected from the group *Vaccinium, Fragaria, Hordeum or* seed oil of *Vaccinium vitis-idaea,* or a mixture thereof, and comprising at least one triglyceride of at least one unsaturated fatty acid, pure seed oil being totally solvent free, not containing any solid content or solid residues and being obtained by supercritical $CO_2$-extraction.

5. A skin care composition according to claim 4, **characterised in that** the amount of the seed oil is 0.01 - 99.99,

typically 0.05 - 50, more typically 0.1 - 25, most typically 1 - 10 weight-% of the weight of the composition.

6. A skin care composition according to claim 4, **characterised in that** said unsaturated fatty acid of the seed oil is selected from the group consisting of linoleic acid and α-linolenic acid.

7. A skin care composition according to any of preceding claims, **characterised in that** the seed oil contains linoleic acid and α-linolenic acids at a ratio of 2:1 to 1:2.

8. A skin care composition according to any of preceding claims, **characterised in that** the seed oil containing 20 - 90 % linoleic acid and α-linolenic acid.

9. A skin care composition according to any of the previous claims, **characterised in that** it is a day cream, a night cream, a face lotion, a body lotion, a body butter, a skin peel, a mask, a shower gel, a sun cream, a sun lotion, an after sun cream or an after sun lotion.

10. A skin care composition according to any of the previous claims, **characterised in that** the dermatologically acceptable additive comprises other lightening ingredients, sunscreen ingredients, preservatives, antioxidants or their combinations.

**Patentansprüche**

1. Verwendung einer Zusammensetzung umfassend ein Samenöl, das von einer Pflanze, ausgewählt aus der Gruppe *Vaccinium, Oxycoccus, Fragaria, Hipphophae, Echium, Hordeum, Avena* oder einem Gemisch davon, abgeleitet ist, umfassend wenigstens ein Triglycerid von wenigstens einer ungesättigten Fettsäure zur Hautaufhellung.

2. Verwendung gemäß Anspruch 1, **dadurch gekennzeichnet, dass** das natürliche Samenöl von *Vaccinium vitis-idaea* ist.

3. Verwendung gemäß einem der vorstehenden Ansprüche, **dadurch gekennzeichnet, dass** die Zusammensetzung eine Tagescreme, eine Nachtcreme, eine Gesichtslotion, eine Körperlotion, eine Körperbutter, ein Haut-Peeling, eine Maske, ein Duschgel, eine Sonnencreme, eine Sonnenlotion, eine Apres-Sun-Creme oder eine Apres-Sun-Lotion ist.

4. Hautpflegezusammensetzung zur Hautaufhellung, umfassend:

   - ein dermatologisch verträgliches Additiv, und
   - ein reines Samenöl, das von einer Pflanze, ausgewählt aus der Gruppe *Vaccinium, Fragaria, Hordeum* abgeleitet ist oder Samenöl von *Vaccinium vitis-idaea* oder einem Gemisch davon, und umfassend wenigstens ein Triglycerid von wenigstens einer ungesättigten Fettsäure, wobei das reine Samenöl vollständig lösungsmittelfrei ist, keine festen Bestandteile oder festen Reste enthält und durch superkritische $CO_2$-Extraktion erhalten wird.

5. Hautpflegezusammensetzung gemäß Anspruch 4, **dadurch gekennzeichnet, dass** die Menge des Samenöls 0,01 - 99,99, typischerweise 0,05 - 50, noch typischer 0,1 - 25, am typischsten 1 - 10 Gewichts-% des Gewichts der Zusammensetzung ist.

6. Hautpflegezusammensetzung gemäß Anspruch 4, **dadurch gekennzeichnet, dass** die ungesättigte Fettsäure des Samenöls aus der Gruppe, bestehend aus Linolsäure und α-Linolensäure, ausgewählt ist.

7. Hautpflegezusammensetzung gemäß einem der vorstehenden Ansprüche, **dadurch gekennzeichnet, dass** das Samenöl Linolsäure und α-Linolensäuren mit einem Verhältnis von 2:1 bis 1:2 enthält.

8. Hautpflegezusammensetzung gemäß einem der vorstehenden Ansprüche, **dadurch gekennzeichnet, dass** das Samenöl 20 - 90 % Linolsäure und α-Linolensäure enthält.

9. Hautpflegezusammensetzung gemäß einem der vorherigen Ansprüche, **dadurch gekennzeichnet, dass** sie eine Tagescreme, eine Nachtcreme, eine Gesichtslotion, eine Körperlotion, eine Körperbutter, ein Hautpeeling, eine

Maske, ein Duschgel, eine Sonnencreme, eine Sonnenlotion, eine Apres-Sun-Creme oder eine Apres-Sun-Lotion ist.

**10.** Hautpflegezusammensetzung gemäß einem der vorherigen Ansprüche, **dadurch gekennzeichnet, dass** das dermatologisch verträgliche Additiv andere Aufhellungsbestandteile, Sonnenschutzbestandteile, Präservative, Antioxidantien oder deren Kombinationen umfasst.

**Revendications**

**1.** Utilisation d'une composition comprenant une huile de graine dérivée d'une plante choisie dans le groupe comprenant *Vaccinium, Oxycoccus, Fragaria, Hipphophae, Echium, Hordeum, Avena,* ou un mélange de celles-ci, comprenant au moins un triglycéride d'au moins un acide gras insaturé pour l'éclaircissement de la peau.

**2.** Utilisation selon la revendication 1, **caractérisée en ce que** l'huile de graine naturelle est de l'huile de graine de *Vaccinium vitis-idaea*.

**3.** Utilisation selon l'une quelconque des revendications précédentes, **caractérisée en ce que** la composition est une crème de jour, une crème de nuit, une lotion pour le visage, une lotion pour le corps, un beurre pour le corps, un gommage, un masque, un gel douche, une crème solaire, une lotion solaire, une crème après-soleil ou une lotion après-soleil.

**4.** Composition de soin pour la peau destinée à l'éclaircissement de la peau comprenant :

- un additif dermatologiquement acceptable, et
- une huile de graine pure dérivée d'une plante choisie dans le groupe comprenant *Vaccinium, Fragaria, Hordeum,* ou une huile de graine de *Vaccinium vitis-idaea,* ou un mélange de celles-ci, et comprenant au moins un triglycéride d'au moins un acide gras insaturé, l'huile de graine pure étant totalement dépourvue de solvant, ne contenant aucune matière sèche ou résidu solide, et étant obtenue par extraction au $CO_2$ supercritique.

**5.** Composition de soin pour la peau selon la revendication 4, **caractérisée en ce que** la quantité de l'huile de graine est de 0,01 à 99,99, typiquement de 0,05 à 50, plus typiquement de 0,1 à 25, le plus typiquement de 1 à 10 % en poids du poids de la composition.

**6.** Composition de soin pour la peau selon la revendication 4, **caractérisée en ce que** ledit acide gras insaturé de l'huile de graine est choisi dans le groupe constitué de 1"acide linoléique et de l'acide $\alpha$-linéolénique.

**7.** Composition de soin pour la peau selon l'une quelconque des revendications précédentes, **caractérisée en ce que** l'huile de graine contient de l'acide linoléique et des acides $\alpha$-linoléniques à un rapport de 2 : 1 à 1 : 2.

**8.** Composition de soin pour la peau selon l'une quelconque des revendications précédentes, **caractérisée en ce que** l'huile de graine contient 20 à 90 % d'acide linoléique et d'acide $\alpha$-linolénique.

**9.** Composition de soin pour la peau selon l'une quelconque des revendications précédentes, **caractérisée en ce qu'**il s'agit d'une crème de jour, d'une crème de nuit, d'une lotion pour le visage, d'une lotion pour le corps, d'un beurre pour le corps, d'un gommage, d'un masque, d'un gel douche, d'une crème solaire, d'une lotion solaire, d'une crème après-soleil ou d'une lotion après-soleil.

**10.** Composition de soin pour la peau selon l'une quelconque des revendications précédentes, **caractérisée en ce que** l'additif dermatologiquement acceptable comprend d'autres ingrédients d'éclaircissement, ingrédients d'écran solaire, conservateurs, antioxydants ou leurs combinaisons.

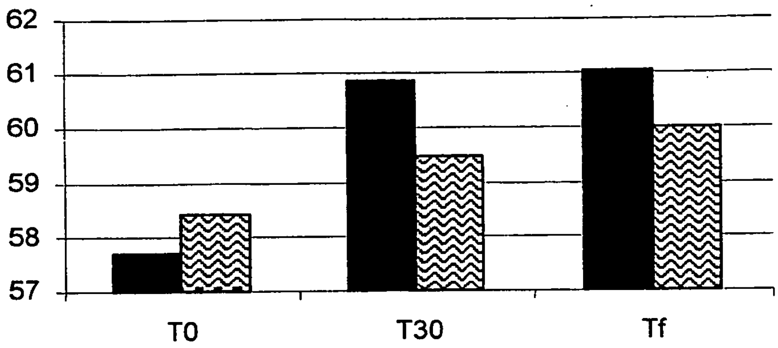


Fig. 1

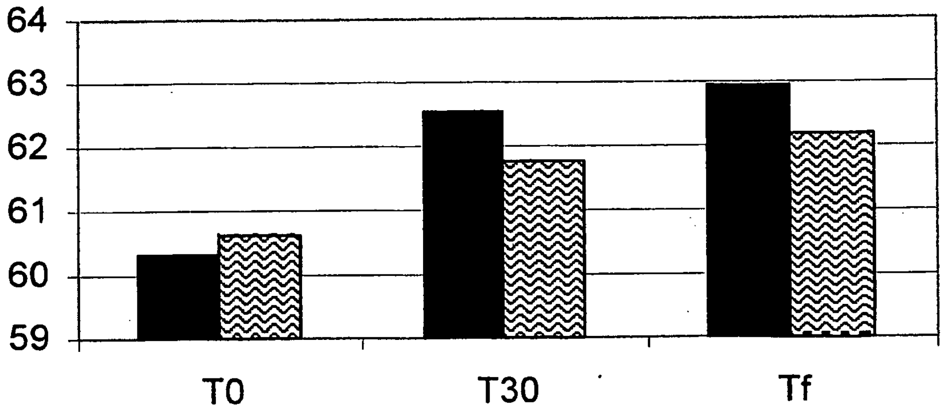


Fig. 2

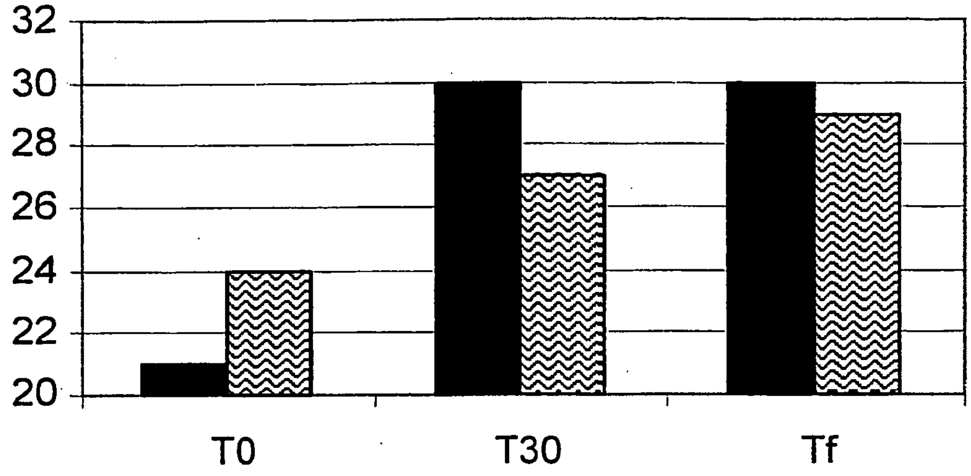


Fig. 3

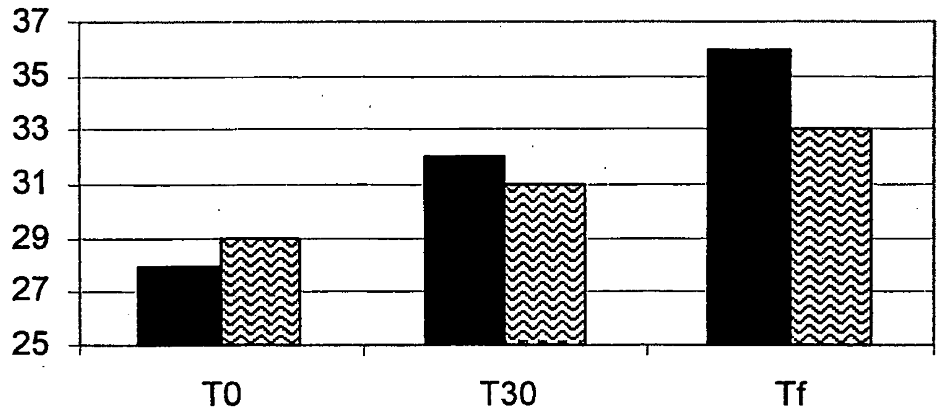


Fig. 4

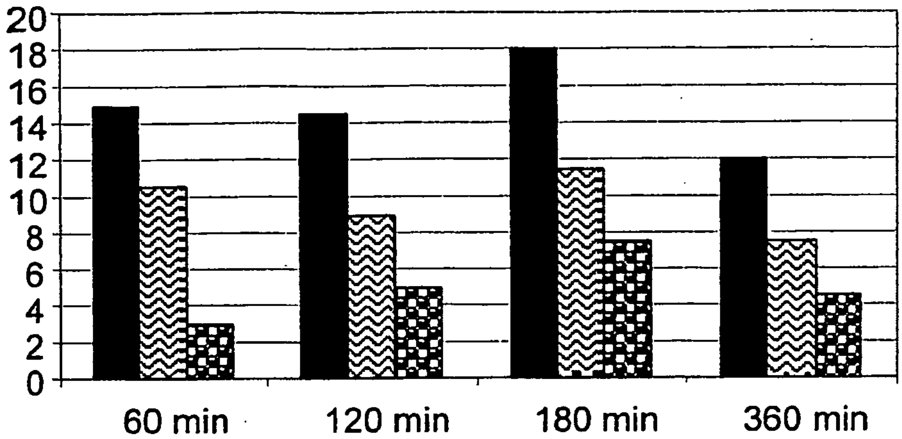


Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 992236 A